# EUROPEAN PATENT APPLICATION

(11) **EP 4 006 157 A2**
(43) Date of publication of application: **01.06.2022**
(21) Application number: 20848144.0
(22) Date of filing: 29.07.2020
(51) Int. Cl.: C12N 15/63, C12N 9/22, C12N 9/78, C12N 15/10, C12N 15/113, C12N 15/90

(54) **CYTOSINE BASE-EDITING COMPOSITION AND USE OF SAME**

(30) Priority: 31.07.2019 KR 20190093518
(71) Applicant: IUCF-HYU (INDUSTRY-UNIVERSITY COOPERATION FOUNDATION HANYANG UNIVERSITY), Seoul 04763 (KR)
(72) Inventor: BAE, Sangsu, Seoul 04763 (KR); KIM, Heon Seok, Seoul 04763 (KR); JEONG, You Kyeong, Seoul 04763 (KR)
(74) Representative: dompatent von Kreisler Selting Werner - Partnerschaft von Patent- und Rechtsanwälten mbB
(86) International application number: PCT/KR2020/010020
(87) International publication number: WO 2021/020884

(57) **Abstract**

The present invention relates to a cytosine base-editing composition, and more specifically, to a cytosine base-editing composition comprising adenine deaminase and CRISPR-associated protein 9 (Cas9), or functional analogs thereof, which has a narrow editing window and thus is capable of accurately editing cytosine in a target sequence with another base.

The present inventors have discovered that the application of the cytosine base-editing composition comprising adenine deaminase and Cas9, or functional analogs thereof, to a target sequence, causes cytosine on a specific motif to be substituted with another base, and that existing adenine base editors (ABEs) are capable of editing not only adenine but also cytosine, and can also have said substitutions occur with higher accuracy compared to existing cytosine base editors (CBEs). Thus, the cytosine base-editing composition according to the present invention is expected to be advantageously used in the treatment of genetic disorders and in research on genetic disorders.

## Description

### [Technical Field]

The present invention relates to a cytosine base-editing composition, and more particularly, to a cytosine base-editing composition, which includes adenine deaminase and a CRISPR-associated protein 9 (Cas9) protein or a functional analogue thereof, and is capable of accurately editing cytosine in a target sequence to another base by having a narrow editing window range.

### [Background Art]

Current genome editing (genome engineering) is performed by a method according to a non-homologous end joining (NHEJ) mechanism which induces insertion-deletion (Indel) mutation by incomplete repair induced in DNA repairing through double-strand breaks at a specific position of a specific gene to be edited, and a method according to homologous recombination occurring in the presence of nearby homologous DNA.

Meanwhile, recently, base editors capable of editing a genome without cutting DNA were developed, and examples thereof include adenine base editors (ABEs) and cytosine base editors (CBEs). CBEs may be constructed by fusing natural cytosine deaminase to dCas9 or nCas9, and edit cytosine to thymine without gene cleavage or insertion of additional donor DNA. On the other hand, it has been reported in academia that ABEs are constructed by fusing artificially-modified adenosine deaminase targeting DNA instead of RNA to Cas9 variants and thus are capable of editing adenine to guanine. The base editors attracted attention as tools to advance research and treatment of intractable genetic diseases, and it is reported that the accuracy of ABEs is being studied for widespread use as a base editing technique.

However, ABEs or CBEs conventionally used for the treatment of intractable genetic diseases have a problem in that efficiency and economic efficiency are lowered by correcting only specific bases, for example, adenine or cytosine.

### [Disclosure]

### [Technical Problem]

The present invention was provided to solve the above problems, and the inventors first identified that cytosine on a specific motif is substituted with another base by applying a cytosine base-editing composition which includes adenine deaminase and a CRISPR associated protein 9 (Cas9) protein or a functional analogue thereof to a target sequence. Based on this, the present invention was completed.

Therefore, the present invention is directed to providing a cytosine base-editing composition, which includes adenine deaminase and a Cas9 protein or a functional analogue thereof.

In addition, the present invention is directed to providing a cytosine base-editing method, which includes bringing the cytosine base-editing composition into contact with a target sequence.

However, technical problems to be solved in the present invention are not limited to the above-described problems, and other problems which are not described herein will be fully understood by those of ordinary skill in the art from the following descriptions.

### [Technical Solution]

To achieve the purposes described above, the present invention provides a cytosine base-editing composition, which includes adenine deaminase and a Cas9 protein or a functional analogue thereof.

In one embodiment of the present invention, the base-editing composition may substitute cytosine with any one base selected from the group consisting of adenine, guanine and thymine.

In another embodiment of the present invention, an editing window of the base-editing composition may be in a range of the base (cytosine) at the fourth position to base (cytosine) at the eighth position from the 5' end of a target sequence.

In still another embodiment of the present invention, the cytosine may be cytosine directly adjacent to thymine located in the 5' direction on a target sequence.

In yet another embodiment of the present invention, the base-editing composition may be any one selected from the group consisting of ABE6.3, ABE7.8, ABE7.9, ABE 7.10 and ABEmax.

In addition, the present invention provides a cytosine base-editing method, which includes bringing the base-editing composition into contact with a target sequence.

### [Advantageous Effects]

The present invention identified that cytosine on a specific motif is substituted with another base, when a cytosine base-editing composition, which includes adenine deaminase and a CRISPR associated protein 9 (Cas9) protein or a functional analogue thereof, is applied to a target sequence, and confirmed that conventional adenine base editors (ABEs) can edit not only adenine, but also cytosine, and compared to conventional cytosine base editors (CBEs), the substitution takes place more accurately. Therefore, it is expected that the cytosine base-editing composition according to the present invention can be effectively used for treatment and research of genetic diseases.

### [Description of Drawings]

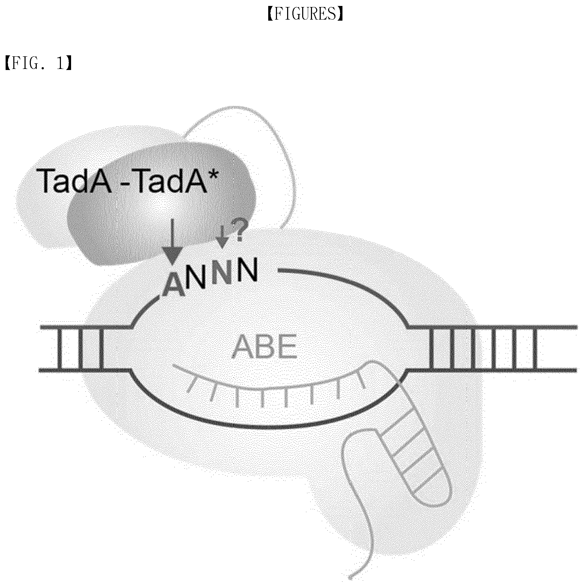

FIG. 1 is a schematic diagram showing the specificity of base editing by a composition according to the present invention.
FIG. 2 shows a result of quantifying a base substitution rate by a composition according to the present invention in a predefined editing window range of a target sequence.
FIG. 3 shows the nucleotide frequency at each position in target regions of FANCF and RNF2 genes. Here, the expected nucleotide frequency is shaded in blue, the substituted nucleotide frequency is shaded in orange, and the protospacer adjacent motif (PAM) sequence is shaded in red.
FIG. 4 shows a result of analyzing base substitution rates induced by compositions according to the present invention (ABE6.3, ABE 7.8, ABE 7.9, ABE 7.10 version) at A4 and C6 sites in FANCF and RNF2 (^{∗} p < 0.1, ^{∗∗} p < 0.001, (n = 3, ± S.D.)).
FIG. 5 shows a result of confirming the influence of UGI on the cytosine substitution rates and proportions of substituted products of compositions according to the present invention (cytosine substitution rate ^{∗∗} p < 0.01. (n = 3, ± S.D.), the proportion of substituted product p < 0.001).

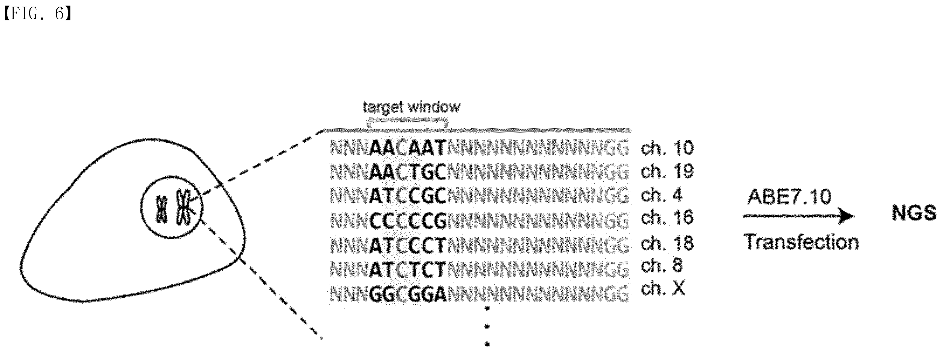

FIG. 6 shows the result of confirming a sequence motif through next-generation sequencing (NGS) in base editing, in which the C^{∗} position is fixed to the 6^{th} position downstream from the 5' end of a target site.
FIG. 7 shows the result of confirming the dependence of a conversion rate of cytosine (C^{∗}) by an upstream base directly adjacent thereto, in which the C^{∗} position is fixed to the 6^{th} position downstream from the 5' end of a target site.
FIG. 8 shows the result of confirming the dependence of a conversion rate of cytosine (C^{∗}) by a downstream base directly adjacent thereto, in which the C^{∗} position is fixed to the 6^{th} position downstream from the 5' end of a target site.
FIG. 9 shows de novo motif analysis data made based on a target sequence in which cytosine editing is induced, in which the C^{∗} position is fixed to the 6^{th} position downstream from the 5' end of a target site.
FIG. 10 shows the result of confirming a base substitution rate at each position in a target sequence of BIRC7 or ABHC12 (^{∗} p < 0.1, ^{∗∗} p < 0.001, (n = 3, ± S.D.)).
FIG. 11 shows the result of confirming a cytosine editing window through NGS.
FIG. 12 shows the result of confirming the dependence of a cytosine substitution rate according to a cytosine position in an editing window.
FIG. 13 shows the result of comparing cytosine editing windows of BE3 and a composition according to the present invention (^{∗} p < 0.05, (n = 2, ± S.D).
FIG. 14 shows the result of confirming cytosine editing efficiency of a composition according to the present invention (ABEmax version) in various cell lines (HEK293T, HeLa, U-2 OS, and K-562) (n = 3, ± S.D.).

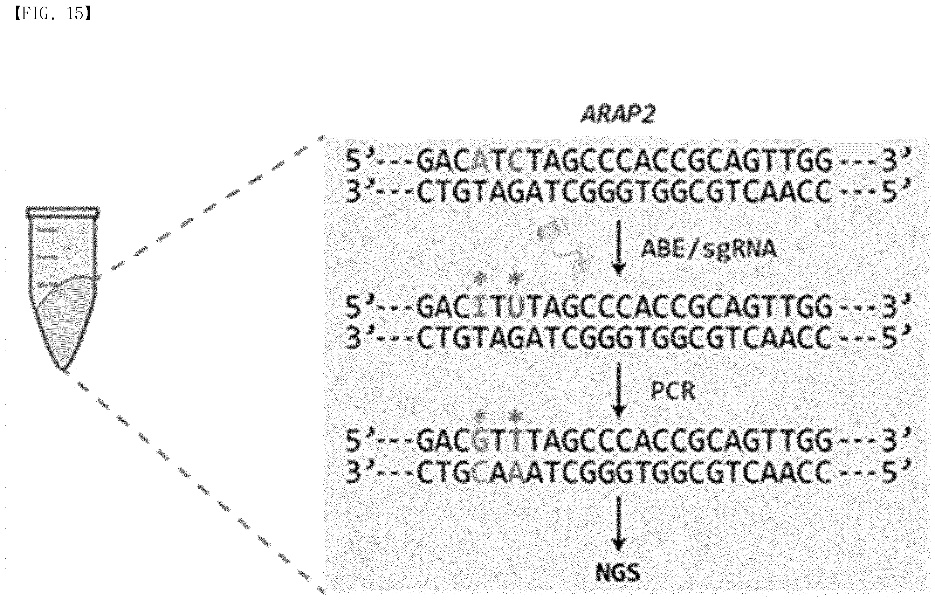

FIG. 15 schematically shows an experimental process for confirming whether a composition according to the present invention performs cytosine editing on a target sequence *in vitro.*

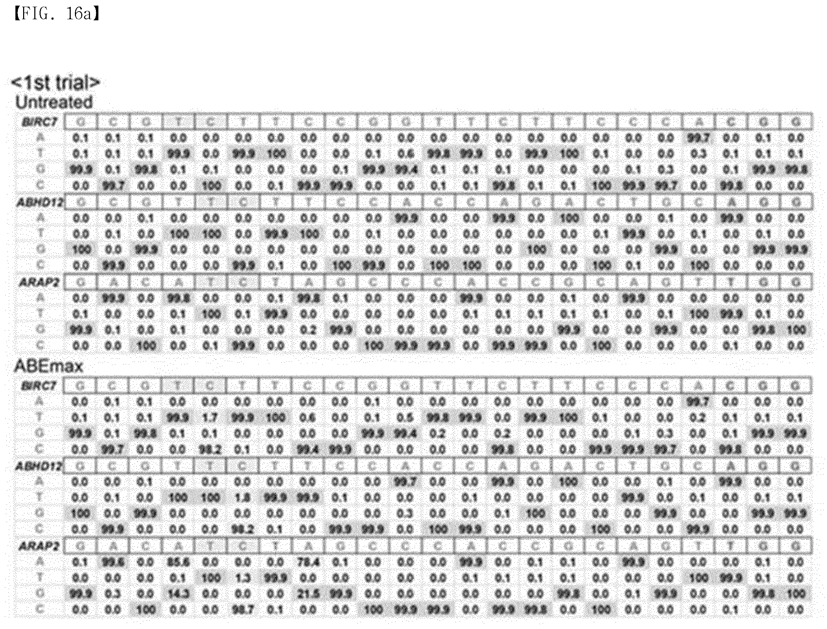

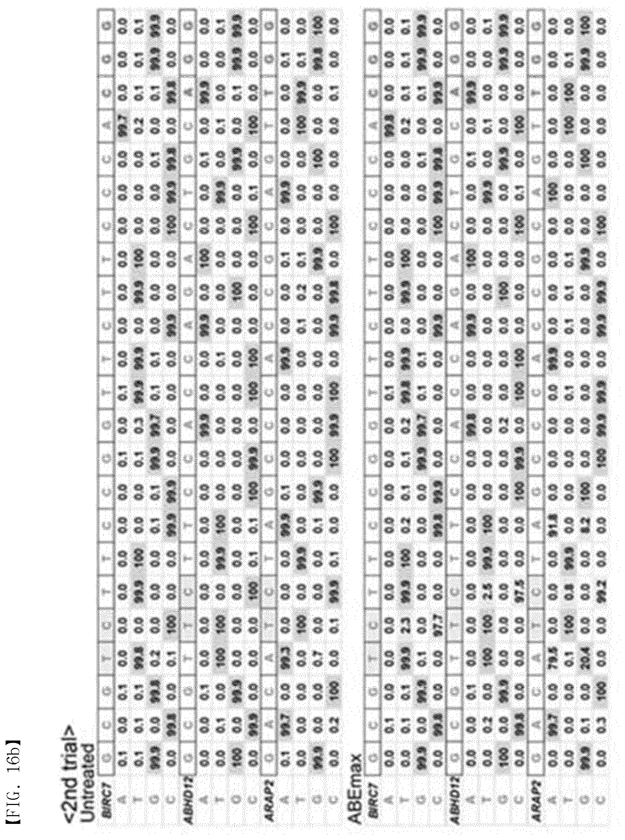

FIGS. 16A and 16B show the results of the first trial and the second trial for confirming whether a composition according to the present invention performs cytosine editing on a target sequence *in vitro.*

### [Modes of the Invention]

Hereinafter, the present invention will be described in detail.

The inventors first identified that ABEs can be used to substitute not only adenine, but also cytosine, on a specific motif with another base, and compared to conventional CBEs, the substitution takes place with higher accuracy, and thus the present invention was completed.

Therefore, the present invention provides a cytosine (C) base-editing composition, which includes adenine deaminase and a CRISPR associated protein 9 (Cas9) protein or a functional analogue thereof.

The term "base editors (BEs)" are single base editing means, and more particularly, are constructed by fusing cytosine deaminase or adenine deaminase to the N-terminus of Cas9 nickase, and thus named a cytosine base editor (CBE) or an adenine base editor (ABE). The BEs do not cause double-strand breaks, ABEs edit adenine to guanine at a specific site, and CBEs edit cytosine to thymine at a specific site.

The term "adenine base editors (ABEs)" are constructed by fusing any natural deaminase (ecTadA) and adenine deaminase variants (ecTadA^{∗}) to the N-terminus of Cas9 nickase in order to edit adenine to guanine, and ABEs include ABE6.3, ABE7.8, ABE7.9, ABE 7.10 and ABEmax according to a version, but the present invention is not limited thereto. The "cytosine (C) base-editing composition including adenine deaminase and a CRISPR associated protein 9 (Cas9) protein or a functional analogue thereof' according to the present invention may be referred to as an "ABE."

The "adenine deaminase" according to the present invention is an enzyme involved in the removal of an amino group and production of hypoxanthine, and it is reported that this enzyme is rarely found in higher animals, but is present in a small amount in muscles of cow, milk, or blood of rats, and in large amounts in the intestines of crayfish and insects. The adenine deaminase includes natural adenine deaminase such as ecTadA, but the present invention is not limited thereto. The adenine deaminase includes variants thereof, such as an ecTadA mutant (ecTadA^{∗}), but the present invention is not limited thereto.

The "CRISPR associated protein 9 (Cas9) protein" according to the present invention is a protein playing an important role in immunological defense of specific bacteria against DNA viruses, and is widely used in genetic engineering applications. Since the key function of the protein is DNA cleavage, the protein can be applied to modify the genome of a cell. Technically, Cas9 is an RNA-guide DNA endonuclease associated with a Clustered Regularly Interspaced Short Palindromic Repeats (CRISPR) adaptive immune system of Streptococcus pyogenes, unwinds a foreign DNA strand to identify a site complementary to a 20-bp spacer region of the guide RNA, and thus, when the DNA is complementary to guide RNA, is considered as invasive DNA and works as a mechanism for cleaving it.

The base-editing composition may substitute cytosine with any one base selected from the group consisting of adenine, guanine and thymine, and preferably, cytosine with guanine or thymine.

The editing window of the base-editing composition may be a range of the cytosine located at the 4^{th} position to the cytosine located at the 8^{th} position from the 5' end of a target sequence, preferably, the cytosine located at the 5^{th} position to the cytosine located at the 7^{th} position from the 5' end thereof, and more preferably, the cytosine located at the 6^{th} position from the 5' end thereof.

The cytosine may be cytosine directly adjacent to thymine located in the 5' direction on a target sequence.

The base-editing composition may be any one selected from the group consisting of ABE6.3, ABE7.8, ABE7.9, ABE 7.10 and ABEmax, but the present invention is not limited thereto.

The inventors confirmed that the ABEs according to the present invention edit cytosine on a specific motif and in an editing window to another base according to a specific example.

More specifically, in one embodiment according to the present invention, it was confirmed that compositions according to the present invention (ABE7.10, ABE 6.3, ABE 7.8 and ABE 7.9 versions) edit cytosine to another base through cytosine deamination (see Example 2).

In another embodiment of the present invention, in terms of cytosine editing induced by a composition of the present invention, as a result of confirming the effect of a nucleotide directly adjacent to cytosine, it was confirmed that cytosine editing efficiency was high in the TC^{∗}N motif (see Example 3).

In still another embodiment of the present invention, as a result of confirming an editing window in which a composition according to the present invention induces cytosine deamination, cytosine editing occurs with the highest frequency at the C6 position, confirming the cytosine editing window is in a range of C5 to C7. Thus, it was confirmed that the cytosine editing window of the composition according to the present invention is narrower than those of CBEs and conventional ABEs (see Example 5).

The above results show that the composition according to the present invention conventionally known to target adenine for editing can be used for cytosine editing, and more specifically show that, to edit cytosine, the composition according to the present invention can be used for cytosine editing by identifying a motif to which the composition specifically binds and an editing window.

Therefore, another aspect of the present invention provides a cytosine base-editing method, which includes bringing the composition according to the present invention into contact with a target sequence.

In the base-editing method according to the present invention, the target sequence may include a target base to be edited, and the target base to be edited may be a base in which a base excluding cytosine and associated with a disease or disorder is converted to cytosine through point mutation, but the present invention is not limited thereto.

Hereinafter, to help in understanding the present invention, exemplary examples will be suggested. However, the following examples are merely provided to more easily understand the present invention, and not to limit the present invention.

### [Examples]

### Example 1. Experimental methods

### 1-1. Conditions for cell culture

HEK293T (ATCC CRL-11268), HeLa (ATCC CLL-2), U-2 OS (ATCC HTB-96) and K-562 (ATCC CCL-243) cells were maintained in DMEM supplemented with10% FBS, 100 µg/mL of streptomycin, 100 units/mL of penicillin and 0.1 mM non-essential amino acids.

### 1-2. Conditions for transfection

1.0 × 10⁵ HEK293T cells were seeded one day before transfection. The cells were transfected with a plasmid expressing ABEs (750 ng) and a sgRNA plasmid (250 ng) using Lipofectamine 2000 (Invitrogen) according to the manufacturer's protocol.

To keep the parallel condition, a 4D karyotype specimen (Lonza) was used for the HEK293T, HeLa, U-2 OS and K-562 cells. The ABEmax expression plasmid (750 ng) and sgRNA plasmid (250ng) were electroporated into 2.0 × 10⁵ of cancer cells according to the manufacturer's protocol.

For respective cell lines, appropriate 216 nucleolus programs (Nucleofector program, CM-130 program for HEK293T, CN-114 program for HeLa, CM-104 program for U-2 OS, and FF-120 program for K-562) were used. Genomic DNA was isolated 72 hours after transfection.

### 1-3. UGI overexpression

A CMV-UGI plasmid based on pCMV-BE3 (Addgene, 221 # 73021) was constructed. To overexpress UGI as well as ABEs, an ABE expression plasmid (500 ng), a sgRNA plasmid (250 ng) and pCMV-UGI (250 ng) were transfected using Lipofectamine 2000. For a negative control, instead of pCMV-UGI, an equal amount of empty pCMV vector was transfected.

### 1-4. In vitro assay for ABE activity

*In vitro* deamination assay was conducted as follows.

A recombinant ABE protein (2000 ng) and sgRNA(1500 ng) were pre-cultured at room temperature for 5 minutes.

Subsequently, genomic DNA (500 ng) isolated and purified from HEK293T cells and 2× buffer was added to the ABE-sgRNA complex. The resulting mixture was prepared to contain 50 mM Tris-HCl, 25 mMKCl, 2.5 mM MgSO₄, 10% glycerol, 2 mM DTT and 10 mM ZnCl₂ and have a final volume of 50 µL.

Afterward, the resulting product prepared by incubating the mixture at 37 °C overnight was purified using a genomic DNA Prep kit (QIAGEN, Catalog #: 69504), followed by PCR amplification.

Finally, the final PCR product was analyzed using targeted deep sequencing.

### 1-5. Targeted deep sequencing

For library preparation using a KAPA HiFi HotStart PCR kit (KAPA Biosystems #: KK2501), a target site was amplified. The library was sequenced using MiniSeq with a TruSeq HT Dual Index system (Illumina).

### 1-6. Data availability

High-throughput sequencing data was deposited in NCBI Sequence Read Archive Database (SRA; https://www.ncbi.nlm.nih.gov/sra) under Accession No. PRJNA525294.

### Example 2. Confirmation of cytosine substitution and deamination by composition according to the present invention

### 2-1. Cytosine substitution by composition according to the present invention

To investigate whether the composition according to the present invention edits a base other than adenine, 22 human endogenous DNA target sites known to have high Cas9 endonuclease activity were identified.

In addition, the inventors assumed that Cas9-mediated base editing induced by the composition according to the present invention occurs at a DNA target site with high Cas9 endonuclease activity at a high level.

A plasmid expressing the composition according to the present invention and a plasmid expressing sgRNA were transfected into human HEK293T cells, and three days after transfection, genomic DNA was isolated.

Afterward, a target site of the composition according to the present invention was amplified, and then treated with an amplicon which was amplified by targeted deep sequencing.

Finally, all base edits induced by the composition at each nucleotide were analyzed in a N3 to N9 range in a sgRNA-binding region of the 5' end target site, which is the "editing window" predefined using a BE analyzer.

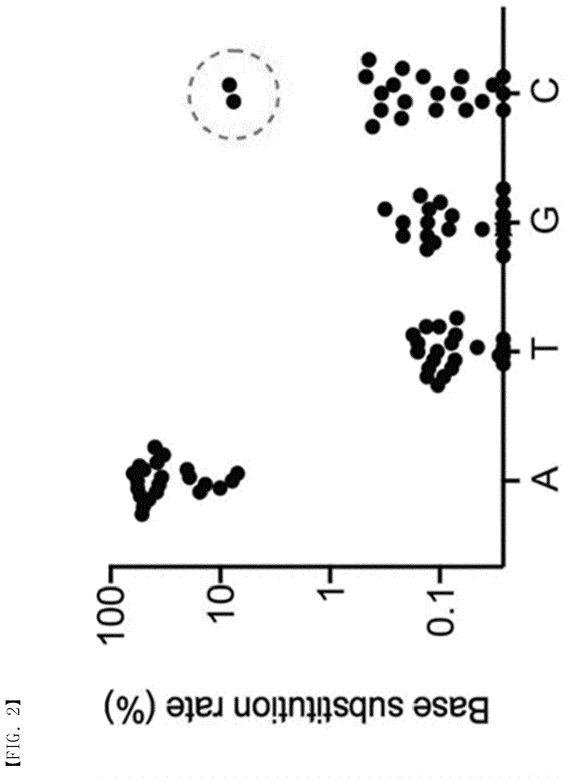

As a result, as shown in FIG. 2, it was confirmed that adenines in the editing window range were edited with an average frequency of 40%.

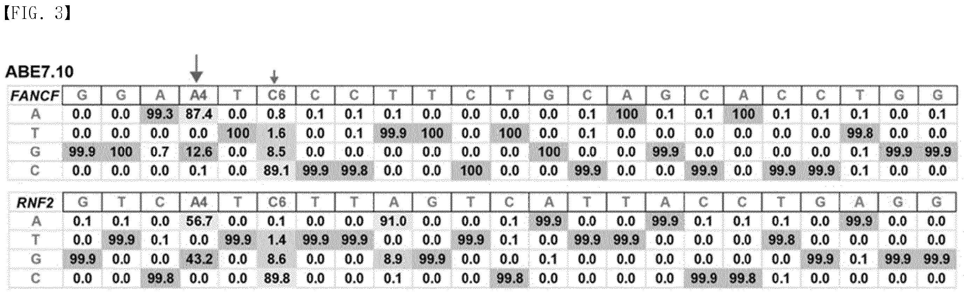

In addition, as shown in FIG. 3, non-standard cytosine base-editing was induced at two target sites of FANCF and RNF2 genes, and deep sequencing data obtained by analyzing it revealed that 10.9% and 10.2% of cytosines in the FANCF and RNF2 target sites, respectively, were converted to other bases by the composition according to the present invention.

Meanwhile, to confirm whether the non-standard cytosine editing varies according to mutation of Cas9 variants in the composition according to the present invention, the experiment was repeated using ABE6.3, ABE 7.8 and ABE 7.9 versions, which are the compositions according to the present invention, other than ABE7.10 (each version represents a different mutation in the adenine deaminase TadA^{∗} enzyme).

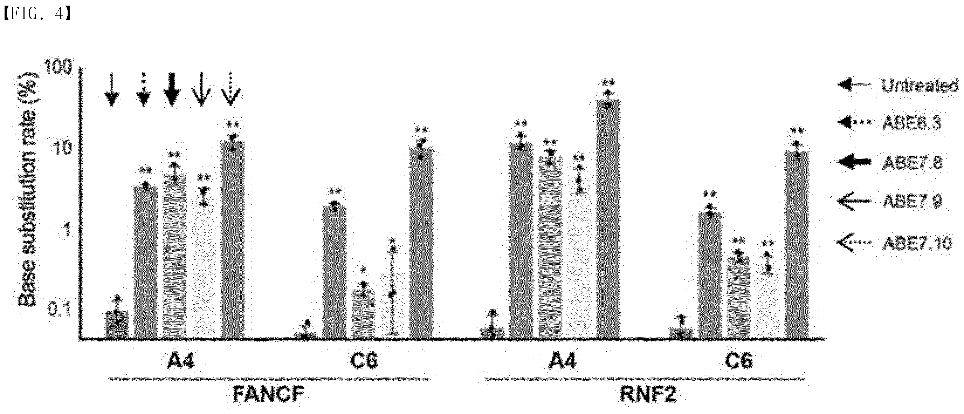

As a result, as shown in FIG. 4, it was confirmed that all of the compositions according to the present invention induce non-standard cytosine base-editing at two target sites.

### 2-2. Confirmation of cytosine deamination by adenine deaminase (TadA^{∗}) of composition according to the present invention

As described above, the composition according to the present invention edits cytosine to various bases such as guanine and thymine.

Meanwhile, when there was a U : G mismatch by cytosine deamination activity of the composition according to the present invention, i) cytosine was substituted with thymine through DNA replication of the mismatched base pair, or ii) DNA repair was performed through uridine cleavage in which uracil was cleaved and then another base was attached.

Therefore, the inventors hypothesized that the adenine deaminase (TadA^{∗}) of the composition according to the present invention deaminates not only adenine, but also cytosine, and to prove the hypothesis, induced substitution of cytosine with thymine by overexpressing a uracil glycosylase inhibitor (hereinafter, UGI) inhibiting uracil cleavage, and applying the composition according to the present invention thereto.

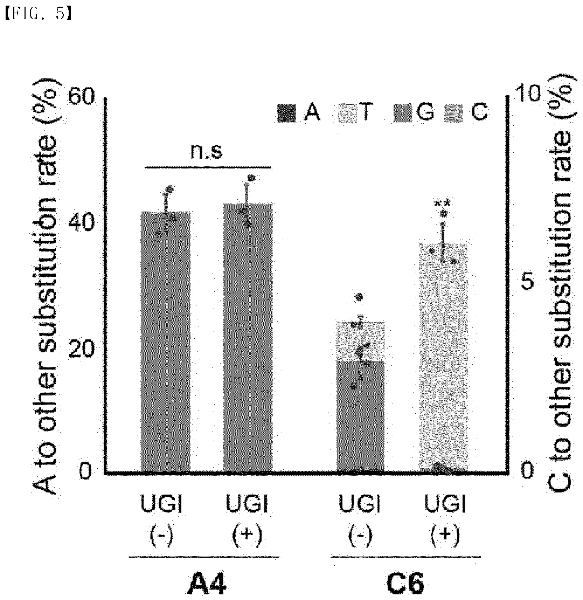

As a result, as shown in FIG. 5, it was confirmed that almost all of cytosines are converted to thymines under an overexpressed UGI condition. This means that the adenine deaminase of the composition according to the present invention directly deaminates cytosine, and it can be seen that the result supports the above hypothesis.

### Example 3. Confirmation of DNA target motif of composition according to the present invention

In terms of the cytosine conversion induced by the composition according to the present invention, to investigate the effect of a nucleotide directly adjacent to cytosine, an endogenous target site containing the NC^{∗} sequence within the editing window range was further identified.

First, using Cas-OFFinder, 28 endogenous target sites located in an exon of a protein-encoding gene including the NC^{∗} sequence near the center of the editing window were selected, and an experiment was conducted to confirm the effect on cytosine conversion according to the type of nucleotide located upstream and directly adjacent to cytosine.

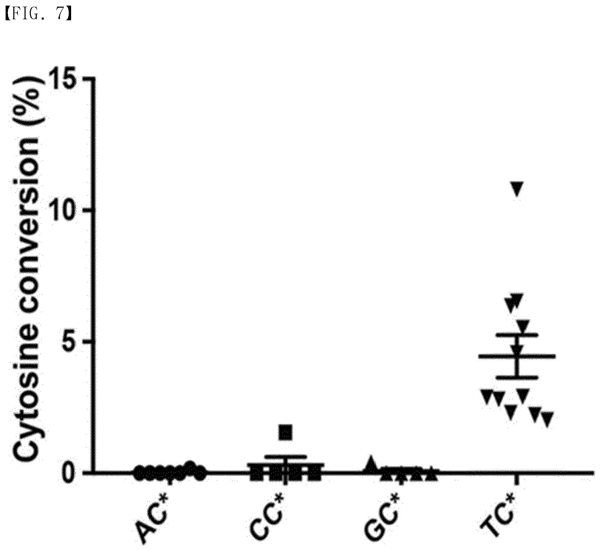

As shown in FIG. 7, the result shows that cytosine directly adjacent to thymine located in the 5' direction of the sequence was efficiently converted by the composition according to the present invention. This result shows a sharp contrast to the conversion rate of cytosine directly adjacent to another nucleotide located in the 5' direction.

In addition, 23 endogenous target sites with standards similar to those described above were additionally identified. The directly adjacent nucleotide located in the 5' direction was fixed to thymine, and then an experiment was conducted to confirm the effect of thymine on cytosine convention according to the type of nucleotide directly adjacent in the 3' direction of cytosine.

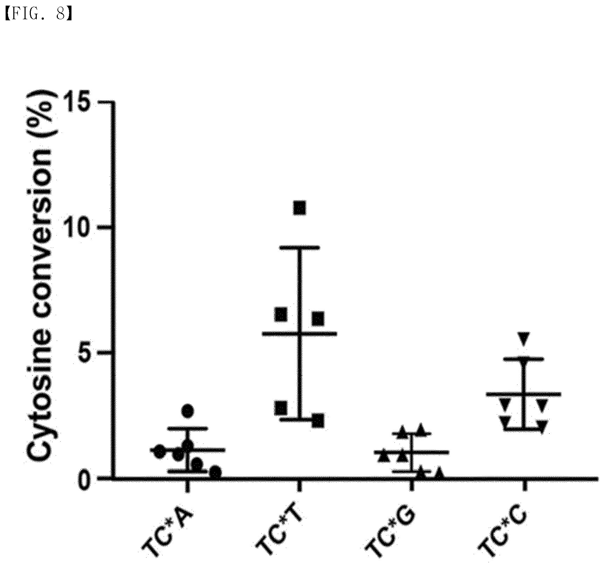

As shown in FIG. 8, the result shows that cytosine directly adjacent to pyrimidine located in the 3' direction in the TC^{∗}N sequence was most efficiently converted, but in most cases, regardless of the type of nucleotide directly adjacent in the 3' direction, base conversion is induced to a measurable level.

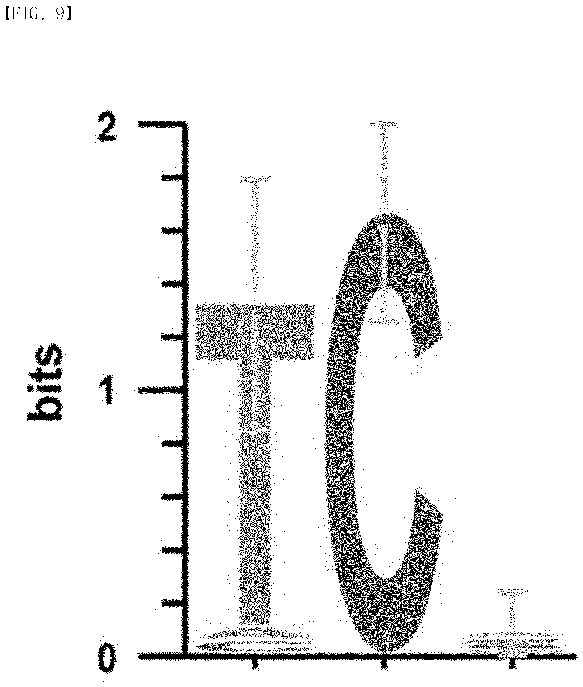

The above results demonstrated that a DNA target motif of cytosine base-editing induced by the composition according to the present invention is TC^{∗}N. This result is consistent with the de novo motif data (see FIG. 9).

### Example 4. Confirmation of independence of cytosine deamination by composition according to the present invention

To confirm whether the cytosine base-editing according to the present invention is affected by deamination of an adjacent adenine, two endogenous target sites which includes the TC^{∗}N motif but not adenine were identified, an experiment was conducted to confirm whether the cytosine deamination induced by the composition according to the present invention is induced independently of adenine deamination.

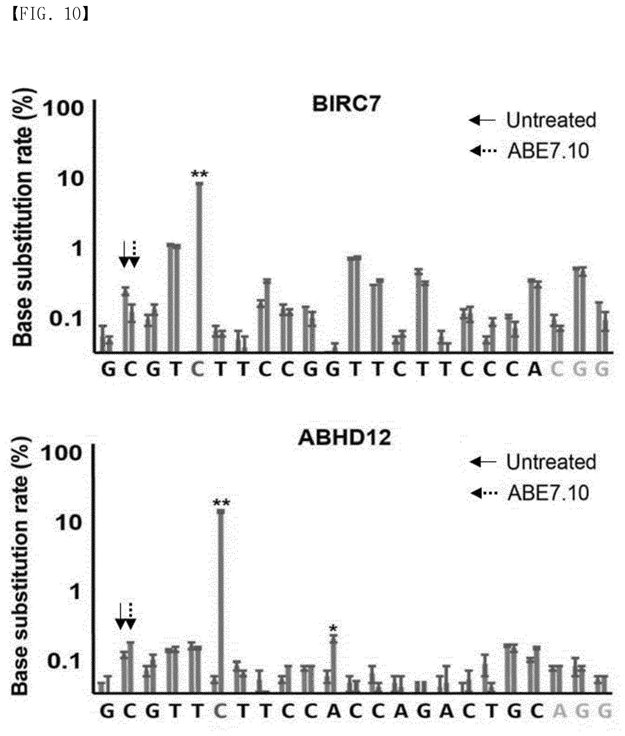

As shown in FIG. 10, the result shows that, even when there is no adenine adjacent to cytosine, cytosine deamination was induced by the composition according to the present invention, resulting in the occurrence of editing.

This result demonstrated that the cytosine deamination induced by the composition according to the present invention is independently performed, not performed as a side effect occurring after adenine deamination.

### Example 5. Confirmation of editing window of composition according to the present invention

The inventors assumed that for the cytosine deamination induced by the composition according to the present invention, a preferable target window can be different from the editing window predefined for adenine deamination.

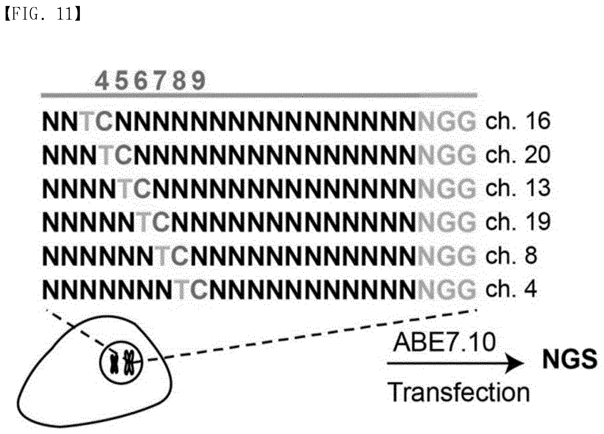

To determine the editing window for efficient cytosine deamination, as shown in FIG. 11, a total of 29 endogenous target sites including the TC^{∗}N motif were selected from various positions in the region of C4 to C9 in the sgRNA-binding region.

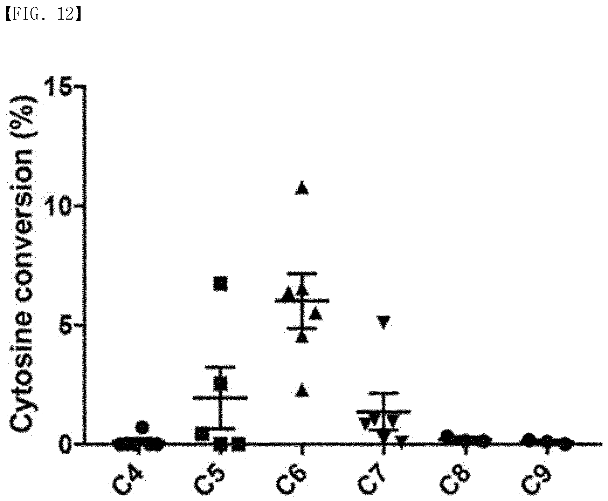

As shown in FIG. 12, the result shows that the cytosine editing induced by the composition according to the present invention occurs with the maximum frequency at the C6 position, and thus the editing window for cytosine deamination is determined to be the C5 to C7 range. This range is narrower than a CBE editing window and a conventional ABE editing window. This confirmed that the composition according to the present invention can more accurately edit cytosine, compared with CBEs.

In addition, at two identical target sites, the cytosine editing activity of the composition according to the present invention was compared with those of conventional CBEs.

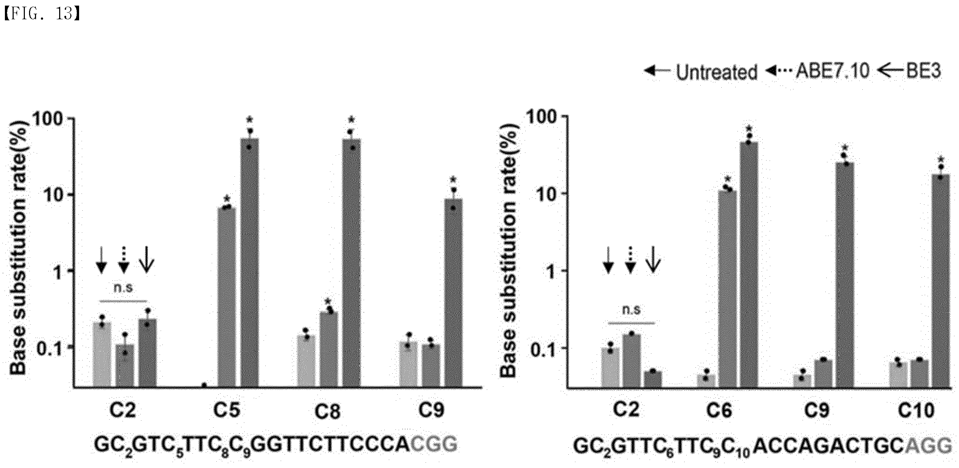

As shown in FIG. 13, the result shows that, while CBEs exhibited higher overall cytosine editing efficiency, the composition according to the present invention more specifically edits cytosine in a narrower editing window.

In addition, to confirm whether the cytosine editing induced by the composition according to the present invention is induced in several other human cell lines, other than the HEK293T cell line, the experiment was repeated with HeLa, U-2 OS and K-562 cell lines.

More specifically, to confirm adenine and cytosine substitution rates at the A4 and C6 positions of a target window in four genes (ABLIM3, CSRNP3, FANCF and RNF2) in the cell lines treated with plasmids expressing the composition according to the present invention, the substitution rate for each nucleotide was measured using targeted deep sequencing.

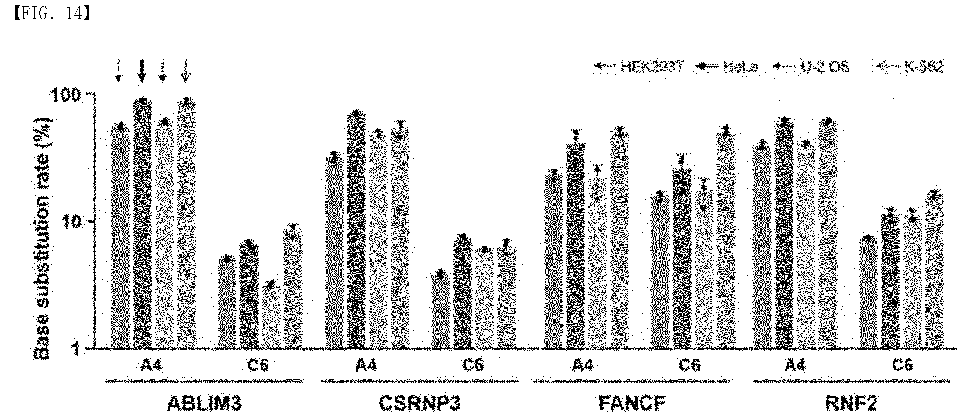

As a result, as shown in FIG. 14, it was confirmed that, in each cell line, cytosine base-editing was induced at a similar level to that in the HEK293T cell line.

It should be understood by those of ordinary skill in the art that the above description of the present invention is exemplary, and the exemplary embodiments disclosed herein can be easily modified into other specific forms without departing from the technical spirit or essential features of the present invention. Therefore, the exemplary embodiments described above should be interpreted as illustrative and not limited in any aspect.

### [Industrial Applicability]

According to the present invention, since ABEs which were previously known to edit only adenine can also be applied to cytosine, ABE editing targets cannot only be increased, but the substitution is also performed with higher accuracy compared to conventional CBEs. Accordingly, the ABEs can be used for cases in which it is difficult to perform accurate editing with conventional CBEs, or can be used for cases in which it is necessary to edit adenine as well as cytosine. Therefore, it is expected that the cytosine base-editing composition according to the present invention will be effectively used for the treatment of and research on various genetic diseases.

## Claims

1. A cytosine (C) base-editing composition, comprising:
adenine deaminase; and
a CRISPR associated protein 9 (Cas9) protein or a functional analogue thereof.

2. The composition of claim 1, wherein the base-editing composition substitutes cytosine (C) with any one base selected from the group consisting of adenine (A), guanine (G) and thymine (T).

3. The composition of claim 1, wherein an editing window of the base-editing composition ranges from the cytosine (C) at the 4^{th} position to the cytosine (C) at the 8^{th} position from the 5' end of a target sequence.

4. The composition of claim 3, wherein the cytosine (C) is cytosine (C) directly adjacent to thymine (T) located in the 5' direction on the target sequence.

5. The composition of claim 1, wherein the base-editing composition is any one selected from the group consisting of ABE6.3, ABE7.8, ABE7.9, ABE 7.10 and ABEmax.

6. A cytosine (C) base-editing method, comprising:
bringing the composition of any one of claims 1 to 5 into contact with a target sequence.
